# EUROPÄISCHE PATENTSCHRIFT

(11) **EP 0 518 175 B1**
(45) Veröffentlichungstag und Bekanntmachung des Hinweises auf die Patenterteilung: **05.01.2000**
(21) Anmeldenummer: 92109247.4
(22) Anmeldetag: 02.06.1992
(51) Int. Cl.: C01B 13/14, C01G 23/047, C01G 49/06, B01J 13/00

(54) **Präparation eines neutralen Titanoxidsols**
Preparation of a neutral titanium oxide sol
Préparation d'un sol neutre d'un oxyde de titanium

(30) Priorität: 14.06.1991 DE 4119719; 06.11.1991 DE 4136512
(43) Veröffentlichungstag der Anmeldung: 16.12.1992
(73) Patentinhaber: MERCK PATENT GmbH, 64271 Darmstadt (DE)
(72) Erfinder: Osterried, Karl, Dr., W-6110 Dieburg (DE); Hechler, Wolfgang, W-6147 Lautertal-Reichenbach (DE); Brückner, Hans-Dieter, Dr., W-6100 Darmstadt 14 (DE); Martin, Roland, Dr. Dipl.-Ing., W-6940 Weinheim (DE)

(56) Entgegenhaltungen:
- EP-A- 0 366 934
- DE-A- 2 637 185
- DE-A- 2 903 263
- US-A- 2 819 177
- US-A- 4 741 781
- COLLOID & POLYMER SCIENCE, Band 262, 1984, Darmstadt (DE); Y. ZHANG, Seiten 723-726

## Beschreibung

Neutrale Titandioxidsole und Verfahren zu ihrer Herstellung sind z. B. beschrieben in EP 0,261,560. Danach wird ein saures Titandioxidsol durch Behandlung mit einem Anionenaustauscherharz neutralisiert. Dabei zeigt das neutralisierte Sol eine Tendenz zur Agglomeration, der dadurch entgegen gewirkt werden kann, indem das Sol mehrere Stunden heftig gerührt wird. Eine andere Möglichkeit zur Vermeidung der Agglomeration besteht darin, daß dem Sol vor oder nach der Behandlung mit dem Anionenaustauscher eine wasserlösliche organische Verbindung zugesetzt wird, wobei hierzu in EP 0,261,560 insbesondere monomere oder polymere Polyalkohole wie z. B. Glycerin, Ethylenglykol oder Polyvinylalkohol vorgeschlagen werden.

Neutrale Metalloxidsole werden in der Technik vielfach verwendet. So können z. B. neutrale Metalloxidsole mit Polymeren gemischt werden oder als Binder für bestimmte Katalysatoren verwendet werden. Neutrale Titandioxid-Sole sind wegen ihrer guten Hautverträglichkeit und ihrer hohen Absorption im UV-Bereich für kosmetische Anwendungen und hier insbesondere für Sonnenschutz-Kosmetika vorgeschlagen worden.

In der U.S. 2,819,177 werden kolloidale TiO₂ - Monohydrat-Dispersionen die aus dem Lösen von TiO₂ in Wasser resultieren, beansprucht.

Die bisher beschriebenen neutralen Metalloxidsole sind jedoch häufig durch eine nicht allen Anforderungen genügende Lagerstabilität gekennzeichnet. Weiterhin weisen die bisherigen Metalloxidsole häufig eine relativ niedrige Konzentration von typischerweise maximal 10 - 25 Gew.% auf; so wird z. B. in EP 0,261,560 ein maximal etwa 25%iges Titandioxidsol durch Erhitzen eines verdünnten, neutralen Titandioxidsols erhalten. Für viele Anwendungen wie z. B. zur Herstellung von Kosmetika sind jedoch hoher konzentrierte Sole erwünscht.

Eine Aufgabe der vorliegenden Erfindung bestand in der Bereitstellung von neutralen Titandioxidsolen, welche die Nachteile herkömmlicher Metalloxidsole nicht oder nur in geringerem Umfang aufweisen. Weiterhin sollte eine Präparation verfügbar gemacht werden, die es dem Anwender gestattet, jederzeit auf einfache Weise Metalloxidsole in der jeweils gewünschten Konzentration selbst herzustellen. Weitere Aufgaben der vorliegenden Erfindung ergeben sich für den Fachmann aus der folgenden detaillierten Beschreibung und aus dem Beispiel.

Es wurde nun gefunden, daß diese Aufgaben durch die Bereitstellung der erfindungsgemäßen Präparationen sowie der erfindungsgemäßen neutralen Titandioxidsolen gelöst werden können.

Gegenstand der Erfindung ist somit ein neutrales TiO₂- Sol mit einer Teilchengröße von 5 - 100 nm erhältlich durch Hydrolyse eines anorganischen Titansalzes in wäßriger Lösung und dessen Überführung in den Solzustand, Stabilisierung des so hergestellten TiO₂ -Sols mit ein oder mehreren Hydroxycarbonsäuren 15 - 45 Gew. % beträgt, bezogen auf das gefriergetrocknete Sol, und Neutralisierung des sauren Gemisches aus Titandioxidsol und/oder Titanoxidhydrat und der Hydroxycarbonsäure und/oder des Hydroxycarbonsäurederivates und gegebenenfalls Gefriertrocknung des stabilisierten Sols und Auflösung des gefriergetrockneten Sols in Wasser oder einer wäßrigen Lösung.

Gegenstand der Erfindung ist weiterhin ein Verfahren zur Herstellung einer derartigen Präparation, dadurch gekennzeichnet, daß durch Hydrolyse eines anorganischen Titansalzes das Titandioxid in den Solzustand überführt wird, ein oder mehrere Hydroxycarbonsäuren und/oder deren Derivate als Stabilisatoren zugesetzt werden, das stabilisierte Sol auf einen pH-Wert zwischen 6 und 8 eingestellt wird, und gegebenenfalls das neutrale Sol gefriergetrocknet und wieder in Wasser oder einer wäßrigen Lösung aufgelöst wird.

Gegenstand der Erfindung sind weiterhin ein konzentriertes Titandioxidsol, erhältlich durch Auflösen einer derartigen Präparation in Wasser oder in einer wäßrigen Lösung sowie die Verwendung dieses Metalloxidsols in kosmetischen Zubereitungen.

Die erfindungsgemäßen Präparationen, welche zur Herstellung eines neutralen Titandioxidsols geeignet sind, enthalten 35 - 75 Gew.%, insbesondere 40 - 70 Gew.% und ganz besonders 50 - 60 Gew.% eines Titandioxids bzw. Titanoxidoxidhydrat,

Ein weiterer Bestandteil der erfindungsgemäßen Präparationen sind eine oder mehrere Hydroxycarbonsäuren bzw. Hydroxycarbonsäurederivate, welche als Stabilisatoren fungieren; dabei sind Zitronen- und/oder Weinsäure bzw. von diesen Säuren abgeleitete Derivate besonders geeignet. Der Massenanteil dieser Stabilisatoren beträgt zwischen 15 und 45 Gew.%, insbesondere zwischen 20 und 40 Gew.% und ganz besonders zwischen 25 und 30 Gew.%.

Daneben können die erfindungsgemäßen Präparationen weitere Bestandteile wie z. B. bei der Herstellung der Präparationen durch Neutralisationsschritte entstehende Salze wie z. B. NaCl, KCl, Na₂SO₄ usw., H₂O oder auch andere oder zusätzliche weitere Bestandteile enthalten.

Der Massenanteil der weiteren Bestandteile an den erfindungsgemäßen Präparationen ist vorzugsweise nicht zu hoch und beträgt insbesondere nicht mehr als 30 Gew.%. Besonders bevorzugt sind Präparationen, bei denen der Massenanteil der weiteren Bestandteile nicht über 20 Gew.% und insbesondere weniger als 15 Gew.% betragt.

Zur Herstellung der erfindungsgemäßen Präparationen geht man aus von einem Titandioxidsol, das nach an sich bekannten Verfahren hergestellt wird. So kann eine wäßrige Lösung eines anorganischen Titansalzes z. B. durch Hydrolyse, die etwa durch Erhitzen bewirkt werden kann, und/oder durch saure Peptisierung und/oder durch Zugabe einer Base oder auch durch weitere Verfahren in den Solzustand überführt werden. In den Beispielen 1 und 2 sind bevorzugte Verfahren zur Herstellung von Titandioxidsol und Eisenoxidsol angegeben; analog können auch Siliciumoxid-, Chromoxid-, Zinnoxid-, Antinomoxid-, Zinkoxid-, Kobaltoxid-, Aluminiumoxid und auch weitere Metalloxidsole hergestellt werden. Die in den Beispielen 1 und 2 angegebenen Verfahren sind jedoch beispielhaft zu verstehen und sollen die Erfindung keinesfalls begrenzen.

Die in den erfindungsgemäß verwendeten Titandioxidsolen enthaltenen Sol-Teilchen weisen typischerweise eine mittlere Größe zwischen 5 und 200 nm und insbesondere zwischen 5 und 100 nm auf. Das Sol kann zur Aufreinigung z. B. in einer Filtrationsanlage mit destilliertem Wasser oder geeigneten Salzlösungen, die nicht zu einer Koagulation des Sols führen, gewaschen werden, wie dies in JP 63-48 358 vorgeschlagen ist.

Zu dem üblicherweise sauren Metalloxidsol wird anschließend eine oder ein Gemisch mehrerer Hydroxycarbonsäuren und/oder Hydroxycarbonsäurederivate hinzugegeben, wobei das Verhältnis aus der Masse der zugesetzten Hydroxycarbonsäuren und/oder Hydroxycarbonsäurederivate typischerweise zwischen 0,2 und 1 beträgt und vorzugsweise zwischen 0,25 und 0,75 und insbesondere zwischen 0,4 und 0,6 liegt. Geeignete Hydroxycarbonsäurederivate sind z. B. Säureamide oder Salze der Hydroxycarbonsäuren. Bevorzugt sind Weinsäure, Zitronensäure und/oder deren Derivate.

Das Gemisch aus Metalloxidsol und einer oder mehreren Hydroxycarbonsäuren bzw. deren Derivaten wird anschließend neutralisiert, wobei hier unter neutral ein pH-Bereich zwischen 5,5 und 8,5 und insbesondere zwischen 6 und 8 verstanden werden soll. Dazu wird zu dem üblicherweise sauren Gemisch aus Metalloxidsol und einer oder mehreren Hydroxycarbonsäuren vorzugsweise eine Base zugegeben wie z. B. NaOH oder KOH. Es ist aber z. B. auch möglich, daß aus dem Gemisch zur Änderung des pH-Wertes das Anion des zur Metalloxidsolherstellung verwendeten anorganischen oder organischen Metallsalzes, z. B. mit Hilfe eines Anionenaustauschers entfernt wird.

Es ist auch möglich, daß die Reihenfolge der beiden bisher beschriebenen Reakionsschritte - Zugabe einer oder mehrerer Hydroxycarbonsäuren bzw. deren Derivate und Neutralisation - vertauscht wird. Wenn die Neutralisation zuerst durchgeführt wird, beobachtet man jedoch in den meisten Fällen eine Koagulation des Metalloxidsols, was die anschließende Umsetzung mit der bzw. den Carbonsäuren und/oder Carbonsäurederivaten erschwert, so daß die oben angegebene Reihenfolge der Reaktionsschritte bevorzugt ist.

Das auf diese Weise erhaltene neutrale und sehr stabile Metalloxidsol wird zur Herstellung der erfindungsgemäßen Präparationen verwendet, wobei verschiedene Verfahren angewendet werden können.

So wird das neutrale Metalloxidsol in einem bevorzugten Verfahren gefriergetrocknet. Man arbeitet üblicherweise zwischen 1 und 10⁻³ mbar und die Trocknungszeiten betragen typischerweise zwischen 2 und 30 Stunden.

In einem anderen, ebenfalls bevorzugten Verfahren wird das neutrale Metalloxidsol mit Hilfe eines Rotationsverdampfers bis zum Trockenen eingeengt. Man arbeitet typischerweise zwischen 5 und 100 mbar und insbesondere zwischen 10 und 25 mbar, und die Temperatur beträgt üblicherweise zwischen 35 und 85 °C und insbesondere zwischen 45 und 60 °C; Abweichungen von diesen bevorzugten Verfahrensbedingungen sind möglich. Es wird vorzugsweise so lange eingeengt, bis keine sichtbare Kondensation am Kühler mehr stattfindet.

In einem anderen, ebenfalls bevorzugten Verfahren wird das neutrale Metalloxidsol sprühgetrocknet. Es können kommerzielle Sprühtrocknungsanlagen verwendet werden; die Eingangstemperatur beträgt üblicherweise zwischen 200 und 500 °C und insbesondere zwischen 200 und 350 °C, und die Ausgangstemperatur liegt typischerweise zwischen 50 und 300 °C und insbesondere zwischen 80 und 220°C.

Der Sprühdruck beträgt typischerweise zwischen 3 und 10 bar. Die genannten Verfahrensbedingungen sind beispielhaft zu verstehen und sollen die Erfindung erläutern, ohne sie zu begrenzen.

Die erfindungsgemäßen Präparationen sind bei luft- und wasserdampfdichter Verpackung außerordentlich lagerstabil; bei photoaktiven Präparationen wie z. B. bei TiO₂-enthaltenden Präparationen empfiehlt sich zudem die Verwendung von Lichtschutzverpackungen.

Der Anwender kann durch einfaches Mischen der erfindungsgemäßen Präparationen mit Wasser oder einer wäßrigen Lösung wieder Metalloxidsole erhalten, wobei er deren Konzentration durch die Menge des zugesetzten Wassers leicht in einem weiten Bereich variieren kann. So können neben verdünnten auch sehr konzentrierte Metalloxidsole von mehr als 30 oder sogar mehr als 40 Gew.% erhalten werden.

Die vollständige und einfache Löslichkeit der erfindungsgemäßen Präparationen in Wasser oder wäßrigen Lösungen ist überraschend, da z. B. durch Gefriertrocknen von sauren Titandioxidsolen oder mit Zitronen- oder Weinsäure versetzten sauren Titandioxidsolen erhaltene Präparationen nicht oder nur teilweise wasserlöslich sind. Die erfindungemäßen Präparationen weisen ein geringeres Transportgewicht auf als Metalloxidsole und sie sind einfach handhabbar. Die durch die Auflösung der Präparationen in Wasser oder wäßrigen Lösungen erhaltenen Metalloxidsole sind außerordentlich lagerstabil; so weisen z. B. durch Weinsäure und/oder Zitronensäure stabiliserte neutrale Sole Standzeiten von weit mehr als 1 Jahr auf. Durch die Gefriertrocknung und das anschließende Lösen der Präparation in Wasser oder einer wäßrigen Lösung ändern sich die Eigenschaften der Metalloxidsole nicht; so bleibt z. B. der Wert für die UV-Extinktion eines Titanioxidsols unbeeinflußt.

Die durch die Auflösung der erfindungsgemäßen Präparationen erhaltenen Metalloxidsole können für vielfältige technische und kosmetische Anwendungen benutzt werden. Besonders bevorzugt ist der Einsatz der durch Auflösen erfindungsgemäßer, TiO₂-haltiger Präparationen hergestellter Titandioxidsole in der Kosmetik. Die erfindungsgemäßen Präparationen können in kosmetische Präparate oder auch nichtkosmetische Zusammensetzungen oder Formulierungen oftmals vorzugsweise auch direkt, eingebracht werden, ohen daß zuvor die Überführung in ein Sol erforderlich wäre. Besonders bevorzugt für kosmetische Anwendungen sind neben TiO₂-haltigen Präparationen weiter eisenoxidhaltige Präparationen, da Eisenoxid im Vergleich zum Titandioxid eine etwa 1,5-fach höhere UV-Extinktion aufweist.

Die aus erfindungsgemäßen Präparationen erhältlichen Metalloxidsole erfüllen die vielfältigen Anforderungen insbesondere bei technischen oder kosmetischen Anwendungen in sehr viel höherem Maße als bisherige Metalloxidsole. Den erfindungsgemäßen Präparationen sowie den daraus erhältlichen Metalloxidsolen kommt somit eine erhebliche wirtschaftliche Bedeutung zu.

Das folgende Beispiel soll die Erfindung erläutern, ohne sie zu begrenzen.

Im vorstehenden und im folgenden bedeuten Prozentangaben Gewichtsprozente und Temperaturen sind in °C angegeben.

### Beispiel 1

### a) Herstellung eines Titandioxidsols

1 l vollentsalztes Wasser wird bei einer Temperatur von 5 °C durch Zugabe von 10%iger HCl auf pH = 1,5 eingestellt. Anschließend werden bei einer Temperatur von 3 - 7 °C 250 ml einer wäßrigen TiCl₄-Lösung (400 g TiCl₄/l) unter starkem Rühren mit einer Dosiergeschwindigkeit von etwa 2 ml/min zugegeben. Durch gleichzeitige Zudosierung eines Ionenaustauschers (Ionenaustauscher II, Handelsprodukt von E. Merck, Darmstadt , Art.-Nr. 4766, Hydroxyl-Form) wird der pH-Wert auf etwa pH = 1,5 gehalten. Danach wird zur Abtrennung des Ionenaustauschers über ein Nylonfilter (Maschenweite etwa 80 µm) abfiltriert. Das Filtrat kann anschließend gegebenenfalls noch feinstfiltriert werden. Man erhält ein etwa 3 - 3,5%iges Titandioxidsol. Durch Eindampfen mit einem Rotationsverdapfer (etwa 30 °C, Druck < 20 mbar) können Titandioxidsole von 10 - 15 Gew.% erhalten werden.

### b) Herstellung einer erfindungsgemäßen TiO₂-haltigen Präparation

200 g eines 12,6 Gew.%igen Titandioxidsols, welches nach dem in a) beschriebenen Verfahren erhalten wird, werden bei pH = 1,5 mit einer Lösung von 12,6 g Zitronensäure in 13 ml H₂O verrührt.

Dabei entsteht ein weißer Brei, der unter Kühlung mit einem Eis-Wasser-Bad mit 32%iger NaOH-Lösung auf pH = 6,7 eingestellt wird; durch die NaOH-Zugabe wird der weiße Brei flüssig und man erhält ein transparentes Sol, welches bei 0,1 mbar 20 Stunden lang gefriergetrocknet wird.

### Beispiel 2

Ein 10%iges, neutrales (pH = 6,7) und mit Zitronensäure stabilisierter TiO₂-Sol wird nach dem in Beispiel 1 a), b) beschriebenen Verfahren hergestellt. Das Sol wird anschließend in einem Rotationsverdampfer bei 10 - 25 mbar und 45 - 60 °C eingeengt, bis keine sichtbare Kondensation am Kühler mehr stattfindet. Man erhalt ein weißes Pulver mit einem Restwassergehalt von etwa 10 - 20 Gew.%.

### Beispiel 3

Ein 9,3%iges, neutrales (pH = 7) und mit Zitronensäure (4,65 Gew.%) stabilisiertes TiO₂-Sol wird nach dem in Beispiel 1 a), b) beschriebenen Verfahren hergestellt.

Das Sol wird anschließend in einer Sprühtrocknungsanlage (transportable Minor-Anlage, Niro Atomizer) unter folgenden (durchschnittlichen) Bedingungen sprühgetrocknet:

| Eingangstemperatur t_{E} [°C] | Ausgangstemperatur t_{A} [°C] | Sprühdruck [bar] | Eigenschaften der erhaltenen Präparation |
|---|---|---|---|
| 245 | 105 | 4,4 | feines, weißes Pulver Wassergehalt 6,1 % gut wasserlöslich |
| 220 | 100 | 4,4 | feines, weißes Pulver Wassergehalt 5,7 % gut wasserlöslich |
| 230 | 110 | 4,4 | feines, weißes Pulver Wassergehalt 4,1 % befriedigend-gut wasserlöslich |

### Beispiel 4

### Zur Herstellung einer Sonnenschutzcreme (O/W) wird Phase A auf 75 °C und Phase B auf 80 °C erhitzt

| | | % |
|---|---|---|
| A | Glyceryl Stearate (und) PEG-100 Stearate | 10,00 |
| | Mineral oil | 25,00 |
| | Cetyl alcohol | 2,00 |
| | Lanolin | 2,00 |
| | Propylparaben | 0,05 |
| B | Titandioxid-Präparation | ad 5,00 TiO₂ |
| | Glycerine | 2,00 |
| | Sorbitol | 3,00 |
| | Methylparaben | 0,15 |
| | Wasser, demineralisiert | ad 100,00 |

Phase B wird anschließend langsam in Phase A eingerührt. Man homogenisiert und läßt unter Rühren auf Raumtemperatur abkühlen. Die Creme kann ggf. anschließend bei 45 °C parfümiert werden.

### Beispiel 5

### Zur Herstellung einer Sonnenschutzcreme (W/O) wird Phase A auf 75 °C und Phase B auf 80 °C erhitzt

| | | % |
|---|---|---|
| A | PEG-1 Glyceryl Oleo Stearate (und) | |
| | Paraffin Wax | 6,00 |
| | Mineral oil subliquidum | 14,50 |
| | Beeswax | 3,00 |
| | Caprylic/Capric Triglyceride | 11,00 |
| | Dimethicone | 2,00 |
| | Tocopherol acetate | 0,50 |
| | Propylparaben | 0,05 |
| B | Titandioxid-Präparation | ad 5,00 TiO₂ |
| | Glycerine | 2,00 |
| | Methylparaben | 0,15 |
| | Wasser, demineralisiert | 10,30 |

Phase B wird anschließend langsam in Phase A eingerührt. Man homogenisiert und läßt unter Rühren auf Raumtemperatur abkühlen. Die Creme kann ggf. anschließend bei 45 °C parfümiert werden.

Die Sonnenschutzcreme weist eine Viskosität von 38.000 mPas (Brookfield RVT, Sp. C, 10 Upm) bei 24 °C auf.

## Patentansprüche

1. Neutrales TiO₂-Sol mit einer Teilchengröße von 5-100 nm erhältlich durch Hydrolyse eines anorganischen Titansalzes in wäßriger Lösung und dessen Überführung in den Solzustand, Stabilisierung des so hergestellten TiO₂-Sols mit ein oder mehreren Hydroxycarbonsäuren oder deren Derivaten, wobei der Anteil an Hydroxycarbonsäuren 15 - 45 Gew.% beträgt, bezogen auf das gefriergetrocknete Sol, und Neutralisierung des sauren Gemisches aus Titandioxidsol und/oder Titanoxidhydrat und der Hydroxycarbonsäure und/oder des Hydroxycarbonsäurederivats und gegebenenfalls Gefriertrocknung des stabilisierten Sols und Auflösung des gefriergetrockneten Sols in Wasser oder einer wäßrigen Lösung.

2. Neutrales TiO₂-Sol nach Anspruch 1, dadurch gekennzeichnet, daß die Hydroxycarbonsäure Weinsäure oder Zitronensäure ist.

3. Neutrales TiO₂-Sol nach Anspruch 1 oder 2, dadurch gekennzeichnet, daß das Hydroxycarbonsäurederivat ein Säureamid oder das Salz einer Hydroxycarbonsäure ist.

4. Verfahren zur Herstellung eines neutralen TiO₂-Sols nach Anspruch 1, dadurch gekennzeichnet, daß durch Hydrolyse eines anorganischen Titansalzes das Titandioxid in den Solzustand überführt wird, ein oder mehrere Hydroxycarbonsäuren und/oder deren Derivate als Stabilisatoren zugesetzt werden, das stabilsierte Sol auf einen pH-Wert zwischen 6 und 8 eingestellt wird, und gegebenenfalls das neutrale Sol gefriergetrocknet und wieder in Wasser oder einer wäßrigen Lösung auflöst wird.

5. Konzentriertes TiO₂-Sol erhältlich durch Auflösen eines neutralen TiO₂-Sols nach einem der Ansprüche 1 - 3 in Wasser oder einer wäßrigen Lösung.

6. Verfahren zur Herstellung eines konzentrierten TiO₂-Sols, dadurch gekennzeichnet, daß ein TiO₂-Sol nach einem der Ansprüche 1 - 3 in Wasser oder gegebenenfalls in einer wäßrigen Lösung aufgelöst wird.

7. Kosmetische Zubereitung enthaltend ein Titandioxidsol nach Anspruch 1.

## Claims

1. Neutral TiO₂ sol having a particle size of 5-100 nm obtainable by hydrolysis of an inorganic titanium salt in aqueous solution and its conversion into the sol state, stabilization of the resulting TiO₂ sol using one or more hydroxycarboxylic acids or derivatives thereof, where the amount of hydroxycarboxylic acids is 15-45% by weight based on the freeze-dried sol, and neutralization of the acidic mixture of titanium dioxide sol and/or titanium oxide hydrate and the hydroxycarboxylic acid and/or hydroxycarboxylic acid derivative and, where appropriate, freeze-drying of the stabilized sol and dissolution of the freeze-dried sol in water or an aqueous solution.

2. Neutral TiO₂ sol according to Claim 1, characterized in that the hydroxycarboxylic acid is tartaric acid or citric acid.

3. Neutral TiO₂ sol according to Claim 1 or 2, characterized in that the hydroxycarboxylic acid derivative is an acid amide or the salt of a hydroxycarboxylic acid.

4. Process for the preparation of a neutral TiO₂ sol according to Claim 1, characterized in that, by hydrolysis of an inorganic titanium salt, the titanium dioxide is converted into the sol state, one or more hydroxycarboxylic acids and/or derivatives thereof are added as stabilizers, the stabilized sol is adjusted to a pH between 6 and 8, and, where appropriate, the neutral sol is freeze-dried and redissolved in water or an aqueous solution.

5. Concentrated TiO₂ sol obtainable by dissolution of a neutral TiO₂ sol according to one of Claims 1-3 in water or an aqueous solution.

6. Process for the preparation of a concentrated TiO₂ sol, characterized in that a TiO₂ sol according to one of Claims 1-3 is dissolved in water or, where appropriate, in an aqueous solution.

7. Cosmetic preparation comprising a titanium dioxide sol according to Claim 1.

## Revendications

1. Sol neutre de TiO₂ présentant une dimension de particules comprise entre 5 et 100 nm obtenu par hydrolyse d'un sel de titane minéral en solution aqueuse et transformation en l'état de sol, stabilisation du sol de TiO₂ ainsi obtenu avec un ou plusieurs acides hydroxycarboxyliques ou dérivés de ceux-ci, la proportion d'acides hydroxycarboxyliques étant comprise entre 15 et 45 % en poids, rapporté au sol lyophilisé, et neutralisation du mélange acide de sol de dioxyde de titane et/ou d'oxyhydrate de titane et de l'acide hydroxycarboxylique et/ou du dérivé de l'acide hydroxycarboxylique et éventuellement lyophilisation du sol stabilisé et dissolution du sol lyophilisé dans l'eau ou dans une solution aqueuse.

2. Sol neutre de TiO₂ selon la revendication 1, caractérisé en ce que l'acide hydroxycarboxylique est l'acide tartrique ou l'acide citrique.

3. Sol neutre de TiO₂ selon la revendication 1 ou 2, caractérisé en ce que le dérivé de l'acide hydroxycarboxylique est un amide ou le sel d'un acide hydroxycarboxylique.

4. Procédé de préparation d'un sol neutre de TiO₂ selon la revendication 1, caractérisé en ce que l'on fait passer le dioxyde de titane à l'état de sol par hydrolyse d'un sel de titane minéral, en ce que l'on ajoute un ou plusieurs acides hydroxycarboxyliques et/ou dérivés de ceux-ci en tant que stabilisants, en ce que l'on ajuste le sol stabilisé à un pH compris entre 6 et 8 et éventuellement en ce que l'on lyophilise le sol neutre et le dissout à nouveau dans l'eau ou dans une solution aqueuse.

5. Sol de TiO₂ concentré obtenu par dissolution d'un sol neutre de TiO₂ selon l'une des revendications 1 à 3 dans l'eau ou dans une solution aqueuse.

6. Procédé de préparation d'un sol de TiO₂ concentré, caractérisé en ce que l'on dissout dans l'eau ou éventuellement dans une solution aqueuse un sol de TiO₂ selon l'une des revendications 1 à 3.

7. Préparation cosmétique contenant un sol de dioxyde de titane selon la revendication 1.
